# EUROPEAN PATENT SPECIFICATION

(11) **EP 3 786 157 B1**
(45) Date of publication and mention of the grant of the patent: **07.08.2024**
(21) Application number: 18916025.2
(22) Date of filing: 25.12.2018
(51) Int. Cl.: C07D 249/06, C07D 401/12, C07D 405/12, C07D 403/10, A61P 35/02, A61K 31/496, A61K 31/5377, A61K 31/551

(54) **PHENYL TRIAZOLE MLL1-WDR5 PROTEIN-PROTEIN INTERACTION INHIBITOR**
PHENYLTRIAZOL-MLL1-WDR5-PROTEIN-PROTEIN-INTERAKTIONSINHIBITOR
INHIBITEUR DE L'INTERACTION PROTÉINE-PROTÉINE MLL1-WDR5 DE PHÉNYLE TRIAZOLE

(30) Priority: 23.04.2018 CN 201810365880
(43) Date of publication of application: 03.03.2021
(73) Proprietor: China Pharmaceutical University, Nanjing, Jiangsu 211198 (CN)
(72) Inventor: YOU, Qidong, Nanjing, Jiangsu 211198 (CN); GUO, Xiaoke, Nanjing, Jiangsu 211198 (CN); LI, Dongdong, Nanjing, Jiangsu 211198 (CN); CHEN, Weilin, Nanjing, Jiangsu 211198 (CN); WANG, Zhihui, Nanjing, Jiangsu 211198 (CN)
(74) Representative: Mewburn Ellis LLP
(86) International application number: PCT/CN2018/123500
(87) International publication number: WO 2019/205687

(56) References cited:
- WO-A1-2017/147700
- WO-A1-2017/147700
- WO-A1-2017/147701
- CN-A- 105 175 284
- CN-A- 105 175 284
- CN-A- 108 715 585
- DONG-DONG LI ET AL: "High-affinity small molecular blockers of mixed lineage leukemia 1 (MLL1)-WDR5 interaction inhibit MLL1 complex H3K4 methyltransferase activity", EUROPEAN JOURNAL OF MEDICINAL CHEMISTRY, vol. 124, 1 November 2016 (2016-11-01), AMSTERDAM, NL, pages 480 - 489, XP055649028, ISSN: 0223-5234, DOI: 10.1016/j.ejmech.2016.08.036
- LI DONG-DONG ET AL: "Structure-based design and synthesis of small molecular inhibitors disturbing the interaction of MLL1-WDR5", EUROPEAN JOURNAL OF MEDICINAL CHEMISTRY, ELSEVIER, AMSTERDAM, NL, vol. 118, 13 April 2016 (2016-04-13), pages 1 - 8, XP029562555, ISSN: 0223-5234, DOI: 10.1016/J.EJMECH.2016.04.032
- MATTHÄUS GETLIK ET AL: "Structure-Based Optimization of a Small Molecule Antagonist of the Interaction Between WD Repeat-Containing Protein 5 (WDR5) and Mixed-Lineage Leukemia 1 (MLL1)", JOURNAL OF MEDICINAL CHEMISTRY, vol. 59, no. 6, 24 March 2016 (2016-03-24), US, pages 2478 - 2496, XP055413735, ISSN: 0022-2623, DOI: 10.1021/acs.jmedchem.5b01630
- BOLSHAN, YURI ET AL.: "Synthesis, Optimization, and Evaluation of Novel Small Molecules as Antagonists of WDR5-MLL Interaction", ACS MEDICINAL CHEMISTRY LETTERS, vol. 4, no. 3, 4 February 2013 (2013-02-04), pages 353 - 357, XP055412290, ISSN: 1948-5875, DOI: 10.1021/ml300467n
- LI, DONGDONG ET AL.: "High-affinity Small Molecular Blockers of Mixed Lineage Leukemia 1 (MLL1)-WDR5 Interaction Inhibit MLL1 Complex H3K4 Methyltransferase Activity", EUROPEAN JOURNAL OF MEDICINAL CHEMISTRY, vol. 124, 20 August 2016 (2016-08-20), pages 480 - 489, XP055649028, ISSN: 0223-5234, DOI: 10.1016/j.ejmech.2016.08.036
- LI, DONGDONG ET AL.: "Structure-based Design and Synthesis of Small Molecular Inhibitors Disturbing the Interaction of MLL1-WDR5", EUROPEAN JOURNAL OF MEDICINAL CHEMISTRY, vol. 118, 13 April 2016 (2016-04-13), pages 1 - 8, XP029562555, ISSN: 0223-5234, DOI: 10.1016/j.ejmech.2016.04.032
- GETLIK, MATTHAUS ET AL.: "Structure-Based Optimization of a Small Molecule Antagonist of the Interaction Between WD Repeat-Containing Protein 5 (WDR5) and Mixed-Lineage Leukemia 1 (MLL1", JOURNAL OF MEDICINAL CHEMISTRY, vol. 59, no. 6, 9 March 2016 (2016-03-09), pages 2478 - 2496, XP055413735, ISSN: 0022-2623, DOI: 10.1021/acs.jmedchem.5b01630

## Description

### FIELD OF THE INVENTION

The present invention relates to the field of pharmaceutical chemistry, and more particularly to phenyl triazole MLL1-WDR5 protein-protein interaction inhibitors, and preparation and medical uses thereof.

### BACKGROUND OF THE INVENTION

Methylation of histones plays a key role in many biological processes and is the focus of study in the epigenetic regulation field. Translocation and re-arrangement of the methyl transferase MLL1 gene for histone H3K4 can lead to mixed lineage leukemia (MLL1, acute myeloid leukemia and acute lymphoid leukemia). MI,L1 gene rearrangement is found in about 10% of leukemia patients. After arrangement, MLL1 gene fuses with other chaperone genes to form fusion genes, and the carcinogenic MLL fusion protein is expressed. The fusion protein can interact with RNA polymerase II (Pol II) related elongation factors to form the super elongation complex (SEC). The complex can lead to abnormal expression of the Hox gene regulated by MLL1 through Pol II, which causes a series of serious consequences to induce MLL leukemia onset.

Chromosomal translocation of MLL1 gene is monoallelic and there is a wildtype MLL1. When the wildtype MLL1 allele is knocked out, the MLL fusion protein alone will not lead to leukemia, and the enzymatic activity of the wildtype MLL1 is necessary for the MI,L1 fusion protein to induce leukemia. Thus, specific inhibition of the enzymatic activity of the wildtype MLL 1 can achieve the effect of treating leukemia.

Catalytic activity on H3K4 methylation by MLL1 alone is very weak and can only result in monomethylation; the enzyme catalytic activity improves greatly upon the formation of the MLL1 core catalytic complex, especially the catalytic activity on H3K4me2. The MLL-C-terminal WIN motif moiety is capable of binding WDR5, RbBP5, Ash2L and DPY30 to form complexes. MLL1 interacts with WDR5 directly through the C-terminal WIN motif moiety, to mediate the interaction between the catalytic domain of MLL1SET and other protein complexes. When WDR5 is knocked out, the level of H3K4me2/3 decreases and the Hox gene expression is downregulated.

Thus, use of small molecule inhibitors to interfere with the protein-protein interaction of MLL1-WDR5 is an effective method to inhibit MLL1 enzymatic activity and downregulate Hox and Meis-1 gene expression to block the progression of leukemia.

### SUMMARY OF THE INVENTION

This invention discloses a small molecule compound that can regulate MLL1-WDR5 protein-protein interaction, which, through interference of MLL1-WDR5 protein-protein interaction, inhibits the enzyme catalytic activity of MLL1, downregulates the methylation level of H3K4 and the gene expression levels of Hox and Meis-1 genes to induce the apoptosis of leukemia cells. Therefore, the compound can be used to treat leukemia. The structure of the compound of the invention is as follows: 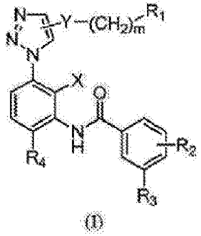 wherein X is hydrogen, methyl, methoxy or halogen;
Y is -CH₂-, -O-, -S-, -CO-, -CH₂O-, -NR₅-, -CONR₆- or -NR₇CO-, wherein R₅, R₆ and R₇ are each independently hydrogen, C₁-C₄ alkyl, C₁-C₄ haloalkyl, phenyl or substituted phenyl, wherein the substituent is halogen, C₁-C₄ alkyl, C₁-C₄ alkoxy, amino, hydroxyl, mercapto, carboxyl, cyano, trifluoromethyl or imidazolyl;
m is 0-6;
R₁ is hydrogen, amino, hydroxyl, mercapto, carboxyl, cyano, -CONH₂, C₁-C₄ alkyl, C₁-C₄ alkoxy, phenyl, substituted phenyl, substituted or unsubstituted nitrogen- or oxygen-containing 3 to 7 membered heterocyclic ring, -NR₈COR₉, -CONR₁₀R₁₁ or -NR₁₀R₁₁, wherein R₈ is hydrogen, C₁-C₄ alkyl, C₁-C₄ haloalkyl, phenyl or substituted phenyl, R₉ is amino, hydroxyl, C₁-C₄ alkyl, C₁-C₄ alkoxy, phenyl or substituted phenyl, and substituted or unsubstituted nitrogen- or oxygen-containing 3 to 7 membered heterocyclic ring, R₁₀ and R₁₁ are independently hydrogen, C₁-C₄ alkyl, phenyl or substituted phenyl, and substituted or unsubstituted nitrogen- or oxygen-containing 3 to 7 membered heterocyclic ring, or R₁₀ and R₁₁ are connected to form nitrogen- or oxygen containing 3 to 7 membered heterocyclic ring, wherein the substituent is halogen, C₁-C₄ alkyl, C₁-C₄ alkoxy, amino, hydroxyl, mercapto, carboxyl, cyano, trifluoromethyl or imidazolyl;
R₂ is halogen, C₁-C₄ alkyl, C₁-C₄ alkoxy, trifluoromethyl, nitro or cyano for disubstitution or trisubstitution;
R₃ is amino, methylamino, aminomethyl, hydroxyl, hydroxymethyl, mercapto or -CONH₂;
R₄ is N-methylpiperazine, 1,2-dimethylpiperazine or N-methylhomopiperazine.

Preferably, X is hydrogen, fluorine, chlorine or methyl.

Preferably, Y is -NR₅-, CONR₆- or -NR₇CO-; and R₅, R₆ and R₇ are each independently hydrogen, methyl, ethyl, propyl, cyclopropyl or isopropyl.

Further preferably, Y is -NR₅-, -CONR₆- or -NR₇CO-; R₅, R₆ and R₇ are each independently substituted phenyl, wherein the substituent is methyl, ethyl, isopropyl, tert-butyl, cyclopropyl, methoxy, cyano, halogen, trifluoromethyl or imidazolyl.

The aforementioned substituted or unsubstituted nitrogen- or oxygen-containing 3 to 7 membered heterocyclic ring is preferably aziridine, azetidine, tetrahydropyrrole, piperidine, hexamethyleneimine, lactam, tetrahydrofuran, tetrahydropyran, morpholine, 1,4-oxazepane, hexahydropyridazine , imidazoline, pyrazolidine, piperazine, wherein the substituent is halogen, methyl, ethyl, phenyl, hydroxyl, amino, hydroxymethyl or aminomethyl.

Preferably, R₁ is -NR₈COR₉, -CONR₁₀R₁₁ or -NR₁₀R₁₁, wherein R₈, R₉, R₁₀ and R₁₁ are C1-C4 alkyl.

Preferably, R₂ is a substituent for trisubstitution and is fluorine, chlorine, bromine, methyl, methoxy, nitro, trifluoromethyl or cyano.

The invention also comprises pharmaceutically acceptable salts and solvates of the compound (I), all of which have the same pharmacological efficacy as the compound (I).

The invention discloses a pharmaceutical composition, which comprises the compound (I) or a pharmaceutically acceptable salt or solvate thereof, as well as one or more pharmaceutically acceptable carriers, diluents and excipients.

The invention also provides the compound of formula (I) or a pharmaceutically acceptable salt or solvate thereof for use in the treatment of diseases mediated by MLL1 through inhibiting MLL1-WDR5 protein-protein interaction, wherein the diseases, such as MLL gene fusion type leukemia can be treated through inhibition of the enzymatic activity of MI,L1.

The dosage of the compound of the invention used clinically is 0.01-1000 mg/day, which can deviate from this range depending on the severity of the disease or dosage forms.

In some embodiments, the compound of formula (I) may contain sufficient basic functional groups to form salts. Representative salts include pharmaceutical inorganic acid salts, such as hydrochloride, hydrobromide and sulfate; pharmaceutically organic acid salts, such as acetate, trifluoroacetate, lactate, succinate, fumarate, maleate, citrate, benzoate, mesylate, p-benzoate and p-toluenesulfonate.

Further, the invention discloses a preparation method of the compounds related to formula (I), including the following steps: 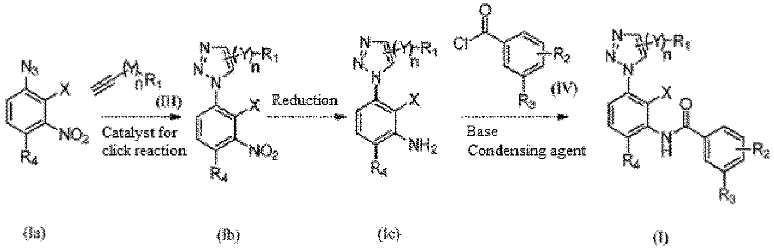 where R₁, R₂, R₃, R₄, X, Y and n are the same as defined above; wherein the intermediate Ia can be obtained through the following synthesis route,

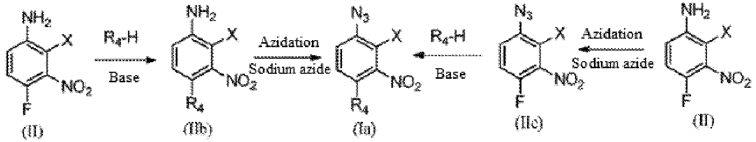

The following are some of the pharmacodynamic tests on the compound of the invention and the test results: MLL1 enzymatic activity is determined by MLL1 and WDR5 protein-protein interaction; MLL1 enzymatic activity affects the methylation level of H3K4. The H3K4 methylation level increases abnormally in MLL fusion type leukemia, and the downstream Hox and Meis-1 gene expression levels are up-regulated abnormally. When MLL1-WDR5 protein-protein interaction is inhibited, MLL1 catalytic activity decreases, H3K4 methylation level decreases, Hox and Meis-1 gene expression levels are downregulated, inhibiting leukemia cell proliferation.

Biphenyl compound DDO-2084 was reported to be a small molecule inhibitor that can inhibit MLL1-WDR5 protein-protein interaction, reduce MLL1 enzyme catalytic activity, and downregulate the expression of Hox and Meis-1 genes (Eur. J. Med. Chem. 2016, 124, 480-489.). In the invention, DDO-2084 is used as a positive control compound.

**Table 1 Inhibitory activity of the compounds of the invention against MLL1-WDR5 protein-protein interaction, and related biological activities**

| Example compound No.^{a} | MLL1-WDR5 PPI inhibitory activity (nM) | Inhibit H3K4 methylation level? | Downregulate *Hox* and *Meis-1* gene expression? |
|---|---|---|---|
| 1 | <80 | Yes | Yes |
| 2 | <50 | Yes | Yes |
| 3 | <10 | Yes | Yes |
| 4 | <10 | Yes | Yes |
| 5 | <10 | Yes | Yes |
| 6 | <10 | Yes | Yes |
| 7 | <10 | Yes | Yes |
| 8 | <10 | Yes | Yes |
| 9 | <50 | Yes | Yes |
| 10 | <10 | Yes | Yes |
| 12 | <80 | Yes | Yes |
| 13 | <80 | Yes | Yes |
| 14 | <10 | Yes | Yes |
| 15 | <10 | Yes | Yes |
| 16 | <10 | Yes | Yes |
| 17 | <50 | Yes | Yes |
| 18 | <50 | Yes | Yes |
| 19 | <50 | Yes | Yes |
| 21 | <80 | Yes | Yes |
| 22 | <10 | Yes | Yes |
| 23 | <50 | Yes | Yes |
| 24 | <50 | Yes | Yes |
| 25 | <50 | Yes | Yes |
| DDO-2084^{b} | 88.7±4.9 | Yes | Yes |

| | | | |
|---|---|---|---|
| ^{a} Refer to specific examples for the structure of the compounds; ^{b} Structure of DDO-2084: 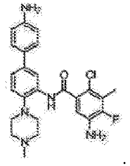 | | | |

As shown in table 1, the compounds of the invention have relatively strong inhibitory activity against MLL1-WDR5 protein-protein interaction.

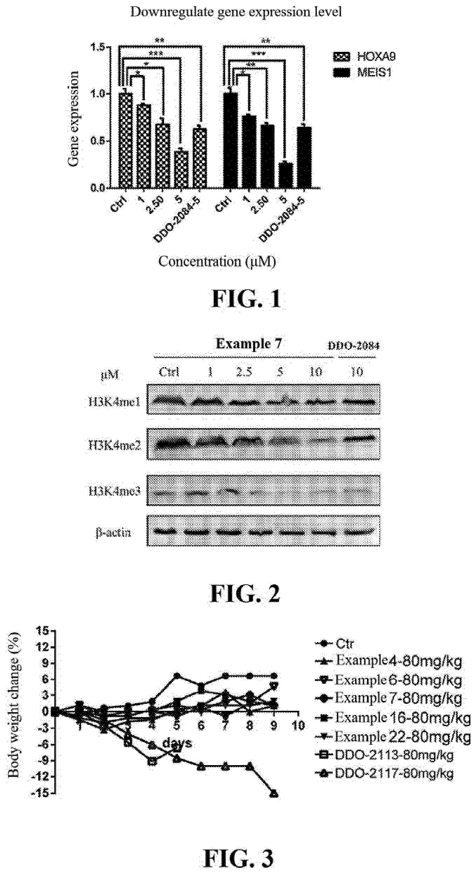

Also, RT-PCR experiments at cellular level were conducted with some of the compounds of the invention, with results listed in table 1 which demonstrates whether some of the compounds of the invention inhibit downstream Hox and Meis-1 gene expression. The results show that all the compounds of the invention with inhibitory activity against MLL1-WDR5 protein-protein interaction can inhibit downstream Hox and Meis-1 gene expression. Inhibition results at cellular level of some of the compounds on the downstream Hox and Meis-1 gene expression are plotted in Fig. 1. As shown in Fig. 1, the inhibitory activity of the compound in Example 7 of the invention at 2.5 µM reached the same level as that of the positive control DDO-2084 at 5 µM on Hoxa9 and Meis-1 gene expression, and Example 7 at 5 µM was superior over DDO-2084 at 5 µM.

In addition, Western-blot experiments at cellular level were conducted with some of the compounds of the invention, with results listed in table 1 which demonstrates whether some of the compounds of the invention inhibit H3K4 methylation level. The results showed that all compounds of the invention with inhibitory activity agaisnt MLL1-WDR5 protein-protein interaction downregulated H3K4 methylation level. Inhibition of MI,L1 catalytic activity at cellular level by some of the compounds is plotted in Fig. 2. As shown in Fig. 2, Example 7 can inhibit MLL1 catalytic activity in a dose-dependent manner to reduce the expression level of H3K4me1/2/3, and it can be seen that the efficacy of Example 7 was better than that of DDO-2084 at the same 10 µM concentration.

In addition, a published article (Eur. J. Med. Chem. 2016, 124, 480-489, referred to as article 1 below) reported a series of biphenyl inhibitors of MLL1-WDR5 protein-protein interaction, and these compounds have a structure of: 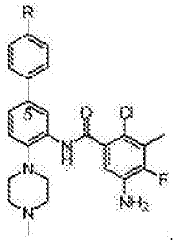 The compounds of the invention differ from these compounds by changing how the two benzene rings are linked, and the 5 position is linked by triazole group instead. With such a change, the compounds of the invention have significantly increased water solubility, reduced toxicity while retaining their original MLL1-WDR5 inhibitory activity. We chose some of the biphenyl compounds in article 1 and some of un-reported biphenyl compounds to test for solubility and toxicity using the same test methods as in the invention simultaneously, and the results are as follows:

**Table 2 Comparison of target activity and solubility of some of the compounds of the invention and some of the compounds in article 1**

| Number and structure of Example compound of the invention | MLL1-WD R5 PP1 inhibitory activity (nM) | Solubility (pH=7.4) µg/mL | Structure of some compounds from article (Eur. J. Med. Chem. 2016, 124, 480-489.) | MLL1-WD R5 PP1 inhibitory activity (nM) | Solubility (pH=7.4) µg/mL |
|---|---|---|---|---|---|
| 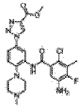 Example 1 | 67.8 | 20 | 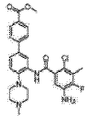 | 70.0 | 1.9 |
| 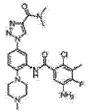 Example 3 | 33.9 | 170 | 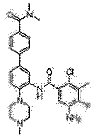 | 73.0 | 21 |
| 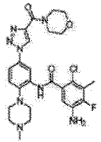 Example 4 | 11.2 | 200 | 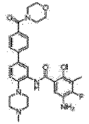 | 55.6 | 27 |
| 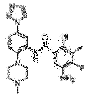 Example 11 | 65.7 | 30 | 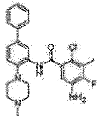 | 47.9 | 3.1 |
| 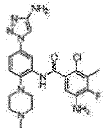 Example 13 | 90.3 | 110 | 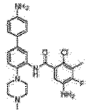 | 88.7 | 9.5 |
| 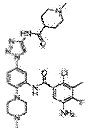 Example 14 | 6.0 | 630 | 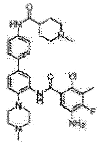 | 6.5 | 54 |
| 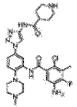 Example 15 | 12.3 | 1235 | 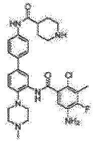 | 8.5 | 125 |
| 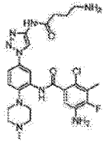 Example 16 | 6.7 | 1303 | 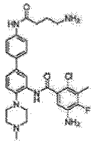 | 7.6 | 110 |

As can be seen from the comparison of data of the compounds in table 2, with the other groups being the same, replacement of the benzene ring by triazole in the invention retained target activity and improved water solubility significantly.

Additionally, subacute toxicity experiments were also conducted to evaluate the safety of some of the compounds of the invention in mouse. Some of the triazole compounds of the invention (Examples 4, 6, 7, 16 and 22) and compounds DDO-2113 and DDO-2117 in article 1 were given by intraperitoneal injection at 80 mg/kg to female balb/c mice, 6 mice per group, for 10 consecutive days to observe mouse survival and average body weight changes. As shown in Fig. 3, there was no death after dosing for 10 days for some of the compounds of the invention (Examples 4, 6, 7, 16 and 22), and there was slight body weight increase, while after dosing with the compounds DDO-2113 and DDO-2117 in the article (Eur. J. Med. Chem. 2016, 124, 480-489.), all mice died after dosing for 5 days with DDO-2113 at 80 mg/kg, and those receiving DOO-2117 had apparent body weight loss. For comparison of post dosing survival and average body weight changes, there was no death but slight body weight increase instead after dosing for 10 days with the phenyl triazole compounds of the invention, while there were deaths and body weight loss after dosing with biphenyl series compounds of DDO-2113 and DDO-2117, indicating the good safety of the phenyl triazole compounds of the invention.

Specifically, DDO-2113 and DDO-2117 are compounds reported in article 1 with the structures below:

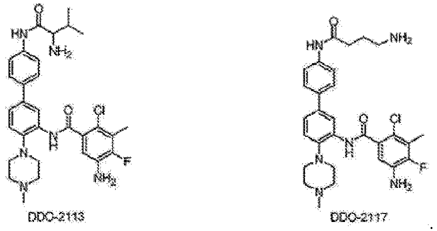

Additionally, experiments on anti-proliferative activity against leukemia cells were conducted with some of the compounds of the invention. Table 3 shows the results of evaluation of the anti-proliferative activity of some of the compounds of the invention against acute leukemia cells, wherein MV4-11 is human acute monocytic leukemia cell, Molm-13 is human acute myeloid leukemia cell, and K562 is human chronic myeloid leukemia cell. Table 3 indicates that the compounds of the invention are effective in inhibiting the proliferation of various leukemia cells.

**Table 3 Anti-proliferative activity of some of the compounds of the invention against leukemia cells**

| Example compound No.^{a} | GI₅₀/µM (MV-411) | GI₅₀/µM (Molm-13) | GI₅₀/µM (K562) |
|---|---|---|---|
| 1 | 8.9±0.3 | 11.8±0.9 | ND |
| 3 | 44.6±1.3 | 22.8±5.5 | >100 |
| 4 | 10.5±2.0 | ND^{b} | 34.4±0.9 |
| 5 | 15.1±1.2 | 13.0±0.6 | 27.5±2.8 |
| 6 | 10.9±0.2 | 8.2±0.6 | 10.4±0.7 |
| 7 | 8.0±1.2 | 9.9±1.9 | 12.9±0.4 |
| 8 | 17.8±3.6 | 11.9±1.5 | 34.1±1.2 |
| 9 | 27.4±2.1 | ND^{b} | 35.8±2.7 |
| 10 | 13.5±1.2 | 13.5±1.2 | 13.5±1.2 |
| 11 | 13.5±1.5 | 13.5±1.5 | 13.5±1.5 |
| 12 | 23.2±3.2 | ND^{b} | ND^{b} |
| 13 | 10.1±1.3 | 9.2±1.4 | 10.5±2.0 |
| 14 | 9.5±1.0 | 11.0±2.0 | 14.1±1.3 |
| 15 | 14.3±1.7 | 18.5±1.9 | 12.9±0.2 |
| 16 | 15.1±0.9 | ND^{b} | 15.0±0.8 |
| 17 | 10.4±1.1 | 7.3±0.8 | 21.3±2.4 |
| 18 | 11.2±1.3 | 15.4±2.2 | ND^{b} |
| 19 | 13.7±1.4 | 16.8±2.0 | 13.5±1.2 |
| 20 | 8.6±0.6 | 10.1±1.3 | 10.5±2.0 |
| 21 | 12.7±0.8 | 7.7±0.9 | 10.5±0.8 |
| 22 | 9.5±0.4 | 11.2±0.8 | 10.9±0.7 |
| 23 | ND^{b} | 17.9±1.6 | 27.7±0.3 |
| 24 | 11.3±0.7 | 12.9±0.9 | 13.5±2.2 |
| 25 | 10.9±0.9 | 8.5±0.7 | 11.3±0.7 |
| DDO-2084 | 17.7±2.3 | ND^{b} | 50.5±5.5 |

| | | | |
|---|---|---|---|
| ^{a} Refer to the specific examples for compound structure; ^{b} ND indicates not detected; | | | |

The phenyl triazole compounds of the invention have strong inhibitory activity against MLL1-WDR5 protein-protein interaction, can reduce the MLL1 catalytic activity of MLL1 at cellular level, downregulate the expression of Hox and Meis-1 genes and induce apoptosis of leukemia cells. Also, the phenyl triazole compounds of the invention exhibit good water solubility and pharmaceutical safety, and can be used for treating leukemia.

### BRIEF DESCRIPTION OF THE DRAWINGS

Fig. 1 shows downregulation of Hoxa9 and Meis-1 gene expression in cells by Example 7 as tested by RT-PCR experiment;
Fig. 2 shows the effects of Example 7 on MLL1 enzyme catalytic activity in cells as tested by Western blot experiment;
Fig. 3 is the toxicity comparison of some compounds of the invention and some compounds in article 1.

### DETAILED DESCRIPTION OF THE INVENTION

### Example 1

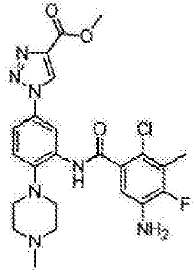

### Methyl 1-(3-(5-amino-2-chloro-4-fluoro-3-methylbenzoylamino)-4 -(4-methylpiperazin-1-yl)phenyl)- 1H-1,2,3-triazol-4-carboxylate (1)

### Preparation of 4-(4-methylpiperazin-1-yl)-3-nitroaniline (IIb):

Compound 4-fluoro-3-nitroaniline (II) (6 g, 38.4 mmol) was dissolved in 50 mL of acetonitrile, N-methylpiperazine (5.8 g, 6.3mL, 57.6 mmol) and N,N-diisopropylethylamine (9.5 mL, 57.6 mmol) were added thereto, and then the reaction mixture was heated to reflux for 12 h. The crude product obtained after spin drying was purified by silica gel column chromatography (dichloromethane : methanol = 20:1) to obtain a red-brown solid (8.9 g, 97.8%). ¹H NMR (300 MHz, DMSO-*d*₆) *δ* 7.06 (d, *J* = 8.6 Hz, 1H), 6.76 (s, 1H), 6.69 (d, *J* = 8.5 Hz, 1H), 5.34 (s, 2H), 2.70 (t, *J=* 4.4 Hz, 4H), 2.27 (br s, 4H), 2.09 (s, 3H). m/z (EI-MS): 259.1 [M + Na]⁺.

### Preparation of 1-(4-azido-2-nitrophenyl)-4-methylpiperazine (Ia):

Compound 4-(4-methylpiperazin-1-yl)-3-nitroaniline (IIb) (4.0 g, 17.0 mmol) was dissolved in 100 mL of 2M/HCl, and cooled to 0°C, to which 10 mL of an aqueous solution of sodium nitrite (1.76 g, 25.5 mmol) was added dropwise, with stirring at 0°C for 30 min. Then to the resultant mixture, 10 mL of an aqueous solution of sodium azide (2.2 g, 34.0 mmol) was added dropwise, and the mixture was stirred at 0°C for 30 min and then at room temperature for 2 h. The product was precipitated using 2 M/NaOH at pH = 9-10, suction-filtrated and oven dried to obtain a red-brown solid (4.0 g, 91.3%). ¹H NMR (300 MHz, DMSO-*d*₆) *δ* 7.48 (d, *J=* 2.2 Hz, 1H), 7.34-7.20 (m, 2H), 2.85 (t, *J=* 4.7 Hz, 4H), 2.31 (t, *J=* 4.8 Hz, 4H), 2.11 (s, 3H). m/z (EI-MS): 261.1 [M-H]⁻.

### Preparation of methyl 1-(4-(4-methylpiperazin-1-yl)-3-nitrophenyl)-1H-1,2,3,-triazol-4-carboxylate (Ib):

Compound 1-(4-azido-2-nitrophenyl)-4-methylpiperazine (Ia) (1.0 g, 3.8 mmol) was dissolved in 50 mL of methanol, to which methyl propiolate (0.96 g, 11.4 mmol), cuprous iodide (0.07 g, 0.38 mmol), N,N-diisopropylethylamine (0.12 mL, 0.76 mmol) were added, the reaction mixture was heated to reflux for 48 h, filtered, concentrated, and slurried with ethyl acetate to obtain a red-brown solid (0.8 g, 61.5%). ¹H NMR (300 MHz, DMSO-*d*₆) *δ* 9.45 (s, 1H), 8.36 (d, *J* = 2.7 Hz, 1H), 8.11-8.01 (m, 1H), 7.42 (d, *J* = 9.1 Hz, 1H), 3.80 (s, 3H), 2.99 (t, *J* = 5.4 Hz, 4H), 2.35 (t, *J* = 5.2 Hz, 4H), 2.13 (s, 3H). m/z (EI-MS): 369.2 [M + Na]⁺.

### Preparation of methyl 1-(3-amino-4-(4-methylpiperazin-1-yl)phenyl-1H-1,2,3-triazol-4-carboxylate (Ic):

Compound methyl 1-(4-(4-methylpiperazin-1-yl)-3-nitrophenyl)-1H-1,2,3-triazol-4-carboxylate (Ib) (3.8 g, 12.0 mmol) was dissolved in 50 mL of methanol, a catalytic amount of Pd/C was added thereto, and hydrogen was introduced. The reaction mixture was stirred at room temperature for 7 h, and concentrated by suction filtration to obtain a pink solid (3.0 g, 78.9%). ¹H NMR (300 MHz, DMSO-*d*₆) *δ* 9.28 (s, 1H), 7.28 (d, *J* = 1.9 Hz, 1H), 7.07 (d, *J* = 1.9 Hz, 2H), 5.15 (s, 2H), 3.90 (s, 3H), 2.87 (t, *J =* 4.5 Hz, 4H), 2.53 (br s, 4H), 2.26 (s, 3H). m/z (ESI-MS): 317.1763 [M + H]⁺.

### Preparation of methyl 1-(3-(5-amino-2-chloro-4-fluoro-3-methylbenzoylamino)-4-(4-methylpiperazin-1-yl)phenyl)-1H-1,2,3-triazol-4-carboxylate (1):

Compound methyl 1-(3-amino-4-(4-methylpiperazin-1-yl)phenyl-1H-1,2,3-triazol-4-carboxylate (Ic) (1.7 g, 5.3 mmol) was dissolved in 100 mL of anhydrous dichloromethane, pyridine (0.43 mL, 5.3 mmol) was added thereto, and then 20 mL of a solution of 2-chloro-3-methyl-4-fluoro-5-nitrobenzoyl chloride (1.6 g, 6.4 mmol) in dichloromethane was added thereto dropwise in an ice-water bath. The reaction mixture was stirred at room temperature for 2 h, and then suction-filtrated and oven-dried to obtain a light yellow solid. The light yellow solid (2.6 g, 4.9 mmol) was dissolved in ethyl acetate, and stannous chloride (5.5 g, 24.4 mmol) was added thereto. The resultant mixture was heated to reflux for 3 h and then cooled down to room temperature, diluted with 100 mL of ethyl acetate, and then neutralized with a saturated sodium bicarbonate solution until no more white gel-like substance appeared. The mixture was suction-filtrated, and the filter cake was washed with ethyl acetate until there was no ultraviolet absorption. The filtrate was extracted with ethyl acetate until there was no ultraviolet absorption. The organic phases were combined, dried over anhydrous sodium sulfate, and concentrated to obtain a crude product which was then slurried with ethyl acetate and suction-filtrated to obtain an off-white solid 1 (2.3 g, 93.9%). ¹H NMR (300 MHz, DMSO-*d*₆) *δ* 9.52-9.45 (m, 2H), 8.69 (s, 1H), 7.73 (dd, *J* = 8.7, 2.7 Hz, 1H), 7.46 (d, *J* = 8.6 Hz, 1H), 6.92 (d, *J* = 9.2 Hz, 1H), 5.53 (s, 2H), 3.92 (s, 3H), 3.00-2.90 (m, 4H), 2.51 (br s, 4H), 2.28 (d, *J=* 2.6 Hz, 3H), 2.24 (s, 3H). (EI-MS): 502.9 [M + H]⁺.

### Example 2

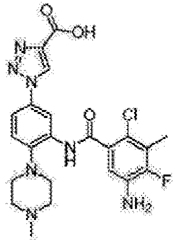

### Preparation of 1-(3-(5-amino-2-chloro-4-fluoro-3-methylbenzoylamino)-4-(4-methylpiperazin-1-yl)phenyl)-1H-1,2,3-triazol-4-carboxylic acid (2):

Compound methyl 1-(3-(5-amino-2-chloro-4-fluoro-3-methylbenzoylamino)-4-(4-methylpiperazin-1-yl)phenyl)-1H-1,2,3-triazol-4-carboxylate (1) (2.3 g, 4.6 mmol) was dissolved in THF, to which a lithium hydroxide solution (1 M, 15 mL) was added. The reaction mixture was stirred at room temperature for 8 h, spin-dried to remove THF, and then acidified with 2 M hydrochloric acid to obtain a white solid (1.7 g, 80.4%). ¹H NMR (300 MHz, DMSO-*d*₆) *δ* 9.52-9.45 (m, 2H), 8.69 (s, 1H), 7.73 (dd, *J* = 8.7, 2.7 Hz, 1H), 7.46 (d, *J* = 8.6 Hz, 1H), 6.92 (d, *J=* 9.2 Hz, 1H), 5.53 (s, 2H), 3.92 (s, 3H), 3.00-2.90 (m, 4H), 2.50 (br s, 4H), 2.28 (d, *J=* 2.6 Hz, 3H), 2.24 (s, 3H). (EI-MS): 488.9 [M + H]⁺.

### Example 3

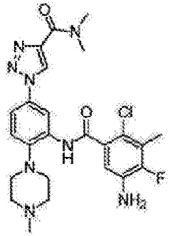

### Preparation of 1-(3-(5-amino-2-chloro-4-fluoro-3-methylbenzoylamino)-4-(4-methylpiperazin-1-yl)phenyl)-N,N-dimethyl-1H-1,2,3-triazol-4-carboxamide (3):

Compound 1-(3-(5-amino-2-chloro-4-fluoro-3-methylbenzoylamino)-4-(4-methylpiperazin-1-yl)phenyl)-1H-1,2,3-triazol-4-carboxylic acid (2) (0.18 g, 0.36 mmol) was dissolved in 10 mL of DMF, to which BOP (0.32 g, 0.72 mmol), trimethylamine (0.10 mL, 0.72 mmol) and dimethylamino hydrochloride (58.7 mg, 0.72 mmol) were added, and stirred at room temperature for 4 h. Then, the reaction mixture was diluted with 50 mL of ethyl acetate, and washed with a saturated sodium chloride solution to remove DMF. The organic phase was dried over anhydrous sodium sulfate, and spin dried to remove the organic solvent to obtain a crude product which was then purified by silica gel column chromatography (dichloromethane : methanol = 50:1) to obtain an off-white solid, with a yield of 78.4%. ¹H NMR (300 MHz, DMSO-*d*₆) *δ* 9.59 (s, 1H), 9.18 (s, 1H), 8.63 (d, *J=* 2.5 Hz, 1H), 7.71 (dd, *J* = 8.6, 2.6 Hz, 1H), 7.42 (d, *J=* 8.6 Hz, 1H), 6.83 (d, *J=* 9.2 Hz, 1H), 5.50 (s, 2H), 3.11 (br s, 10H), 3.02 (s, 3H), 2.70-2.69 (m, 4H), 2.24 (d, *J* = 2.5 Hz, 3H). (EI-MS): 515.9 [M + H]⁺.

### Example 4

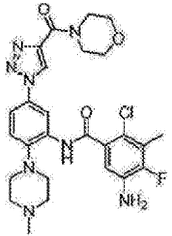

### Preparation of 5-amino-2-chloro-4-fluoro-3-methyl-N-(2-(4-methylpiperazin-1-yl)-5-(4-(morpholin-4-carbo nyl)-1H-1,2,3-triazol-1-yl)phenyl)benzamide (4):

This example was prepared according to the method in Example 3, wherein dimethylamino hydrochloride was replaced with morpholine. An off-white solid was obtained with a yield of 67.5%.¹H NMR (300 MHz, DMSO-*d*₆) *δ* 9.61 (s, 1H), 9.24 (s, 1H), 8.64 (d, *J* = 2.5 Hz, 1H), 7.73 (dd, *J* = 8.6, 2.6 Hz, 1H), 7.44 (d, *J* = 8.7 Hz, 1H), 6.86 (d, *J* = 9.2 Hz, 1H), 5.52 (s, 2H), 4.06 (s, 2H), 3.68 (s, 6H), 3.12 (br s, 8H), 2.69 (s, 3H), 2.26 (d, *J* = 2.6 Hz, 3H). (EI-MS): 558.9 [M + H]⁺.

### Example 5

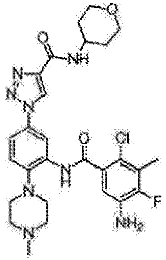

### Preparation of 1-(3-(5-amino-2-chloro-4-fluoro-3-methylbenzoylamino)-4-(4-methylpiperazin-1-yl)phenyl)-N-(tetrahydro-2H-pyran-4-yl)-1H-1,2,3-triazol-4-carboxamide (5):

This example was prepared according to the method in Example 3, wherein dimethylamino hydrochloride was replaced with 4-aminotetrahydropyran. An off-white solid was obtained with a yield of 82.9%. ¹H NMR (300 MHz, DMSO-*d*₆) *δ* 9.52 (s, 1H), 9.26-9.24 (m, 1H), 8.65 (s, 2H), 7.71 (d, *J=* 8.7 Hz, 1H), 7.42 (d, *J=* 9.4 Hz, 1H), 6.89 (s, 1H), 5.53 (s, 2H), 4.06 (s, 1H), 3.9-3.86 (m, 2H), 2.99 (s, 6H), 2.71 (s, 4H), 2.38 (s, 3H), 2.25 (s, 3H), 1.71 (s, 4H). (EI-MS): 572.0 [M + H]⁺.

### Example 6

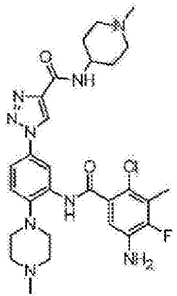

### Preparation of 1-(3-(5-amino-2-chloro-4-fluoro-3-methylbenzoylamino)-4-(4-methylpiperazin-1-yl)phenyl)-N-(1-methylpiperidin-4-yl)-1H-1,2,3-triazol-4-carboxamide (6):

This example was prepared according to the method in Example 3, wherein dimethylamino hydrochloride was replaced with 4-amino-1-methylpiperidine. An off-white solid was obtained with a yield of 49.9%. ¹H NMR (300 MHz, DMSO-*d*₆) *δ* 9.48 (s, 1H), 9.22 (s, 1H), 8.65 (d, *J=* 2.6 Hz, 1H), 8.52 (d, *J=* 8.2 Hz, 1H), 7.70 (dd, *J=* 8.5, 2.7 Hz, 1H), 7.42 (d, *J* = 8.6 Hz, 1H), 6.89 (d, *J* = 9.2 Hz, 1H), 5.54 (s, 2H), 3.78 (s, 1H), 2.92 (t, *J* = 4.6 Hz, 4H), 2.82-2.78 (m, 2H), 2.26 (d, *J=* 2.7 Hz, 3H), 2.22 (s, 3H), 2.19 (s, 3H), 2.07-1.86 (m, 4H), 1.75-1.66 (m, 4H). (EI-MS): 585.0 [M + H]⁺.

### Example 7

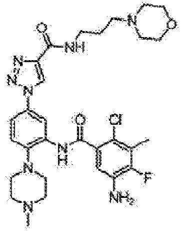

### Preparation of 1-(3-(5-amino-2-chloro-4-fluoro-3-methylbenzoylamino)-4-(4-methylpiperazin-1-yl)phenyl)-N-(3-morpholinopropyl)-1H-1,2,3-triazol-4-carboxamide (7):

This example was prepared according to the method in Example 3, wherein dimethylamino hydrochloride was replaced with N-(3-aminopropyl)morpholine. An off-white solid was obtained with a yield of 94.2%. ¹H NMR (300 MHz, DMSO-*d*₆) *δ* 9.51 (s, 1H), 9.22 (s, 1H), 8.85 (t, *J* = 5.8 Hz, 1H), 8.66 (s, 1H), 7.71 (dd, *J* = 8.6, 2.7 Hz, 1H), 7.43 (d, *J* = 8.8 Hz, 1H), 6.89 (d, *J* = 9.2 Hz, 1H), 5.53 (s, 2H), 3.62 (t, *J =* 4.6 Hz, 4H), 2.98-2.97 (m, 4H), 2.63 (s, 4H), 2.54 (s, 2H), 2.46-2.36 (m, 6H), 2.33 (s, 3H), 2.26 (d, *J* = 2.6 Hz, 3H), 1.74-1.70 (m, 2H). (EI-MS): 615.1 [M + H]⁺.

### Example 8

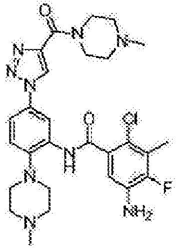

### Preparation of 5-amino-2-chloro-4-fluoro-3-methyl-N-(2-(4-methylpiperazin-1-yl)-5-(4-(4-methylpiperazin-1-carbonyl)-1H-1,2,3-triazol-1-yl)phenyl)benzamide (8):

This example was prepared according to the method in Example 3, wherein dimethylamino hydrochloride was replaced with N-methylpiperazine. An off-white solid was obtained with a yield of 94.2%. ¹H NMR (300 MHz, DMSO-*d*₆) *δ* 9.51 (s, 1H), 9.22 (s, 1H), 8.85 (t, *J* = 5.8 Hz, 1H), 8.66 (s, 1H), 7.71 (dd, *J* = 8.6, 2.7 Hz, 1H), 7.43 (d, *J* = 8.8 Hz, 1H), 6.89 (d, *J* = 9.2 Hz, 1H), 5.53 (s, 2H), 3.62 (t, *J* = 4.6 Hz, 4H), 2.98-2.97 (m, 4H), 2.63 (s, 4H), 2.54 (s, 2H), 2.46-2.36 (m, 6H), 2.33 (s, 3H), 2.26 (d, *J =* 2.6 Hz, 3H), 1.74-1.70 (m, 2H). (EI-MS): 571.0 [M + H]⁺.

### Example 9

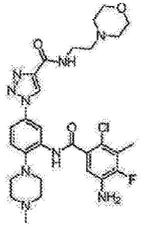

### Preparation of 1-(3-(5-amino-2-chloro-4-fluoro-3-methylbenzoylamino)-4-(4-methylpiperazin-1-yl)phenyl)-N-(2-morpholinoethyl)-1H-1,2,3-triazol-4-carboxamide (9):

This example was prepared according to the method in Example 3, wherein dimethylamino hydrochloride was replaced with N-(2-aminoethyl)morpholine. An off-white solid was obtained with a yield of 72.5%. ¹H NMR (300 MHz, DMSO-*d*₆) *δ* 9.51 (s, 1H), 9.24 (s, 1H), 8.66 (d, *J* = 2.4 Hz, 1H), 8.56 (t, *J* = 5.8 Hz, 1H), 7.71 (dd, *J* = 8.7, 2.6 Hz, 1H), 7.43 (d, *J=* 8.7 Hz, 1H), 6.89 (d, *J=* 9.2 Hz, 1H), 5.54 (s, 2H), 3.58 (t, *J=* 4.6 Hz, 4H), 3.44 (s, 2H), 2.96 (t, *J=* 4.8 Hz, 4H), 2.59 (s, 4H), 2.54 (s, 2H), 2.44 (s, 4H), 2.30 (s, 3H), 2.26 (d, *J* = 2.6 Hz, 3H). (EI-MS): 601.0 [M + H]⁺.

### Example 10

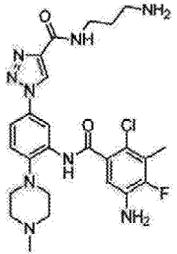

### Preparation of 1-(3-(5-amino-2-chloro-4-fluoro-3-methylbenzoylamino)-4-(4-methylpiperazin-1-yl)phenyl)-N-(3-aminopropyl)-1H-1,2,3-triazol-4-carboxamide (10):

This example was prepared according to the method in Example 3, wherein dimethylamino hydrochloride was replaced with 1,3-propylene diamine. An off-white solid was obtained with a yield of 64.5%. ¹H NMR (300 MHz, DMSO-*d*₆) *δ* 8.85 (s, 1H), 8.70 (s, 1H), 8.45 (d, *J* = 2.1 Hz, 1H), 7.36 (dd, *J* = 7.5, 2.0 Hz, 1H), 7.25 (s, 1H), 6.84 (d, *J* = 7.5 Hz, 1H), 6.78 (d, *J =* 5.7 Hz, 1H), 4.13 (s, 2H), 3.25-3.19 (m, 6H), 2.98 (t, *J =* 4.9 Hz, 4H), 2.67-2.58 (m, 5H), 2.39 (s, 3H), 2.19-2.13 (m, 2H), 1.14 (s, 2H). (EI-MS): 545.0 [M + H]⁺.

### Example 11

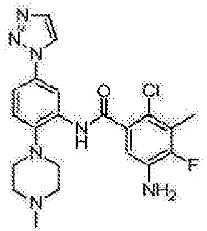

### 5-amino-2-chloro-4-fluoro-3-methyl-N-(2-(4-methylpiperazin-1-yl)-5-(1H-1,2,3-triazol-1-yl)phenyl)benzamide (11)

This example was prepared according to the method in Example 1, wherein methyl propiolate was replaced with trimethylsilylacetylene. An off-white solid was obtaining through three reaction steps. The yield of the three steps is 23.8%. ¹H NMR (300 MHz, DMSO-*d*₆) *δ* 8.76-8.68 (m, 2H), 8.49 (d, *J=* 2.1 Hz, 1H), 8.18 (d, *J=* 7.5 Hz, 1H), 7.27 (dd, *J* = 7.5, 2.0 Hz, 1H), 6.82 (d, *J=* 7.5 Hz, 1H), 6.75 (d, *J=* 5.7 Hz, 1H), 4.15 (s, 2H), 3.20 (t, *J=* 5.1 Hz, 4H), 2.98 (t, *J=* 5.0 Hz, 4H), 2.60 (s, 3H), 2.39 (s, 3H). (EI-MS): 444.9 [M + H]⁺.

### Example 12

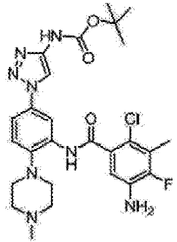

### Tert-butyl(1-(3-(5-amino-2-chloro-4-fluoro-3-methylbenzoylamino)-4-(4-methylpiperazi n-1-yl)phenyl)-1H-1,2,3-triazol-4-yl)carbamate (12)

This example was prepared according to the method in Example 1, wherein methyl propiolate was replaced with tert-butyl ethynyl carbamate. An off-white solid was obtained through three reaction steps. The yield of the three steps is 20.1%.¹H NMR (300 MHz, DMSO-*d*₆) *δ* 8.70 (s, 1H), 8.59 (d, *J* = 2.0 Hz, 1H), 8.08 (s, 1H), 7.25 (dd, *J* = 7.5, 2.0 Hz, 1H), 7.18 (s, 1H), 6.80 (dd, *J* = 19.7, 6.6 Hz, 2H), 4.17 (s, 2H), 3.20 (t, *J* = 5.1 Hz, 4H), 2.98 (t, *J=* 5.1 Hz, 4H), 2.60 (s, 3H), 2.39 (s, 3H), 1.50 (s, 9H).(EI-MS): 560.0 [M + H]⁺.

### Example 13

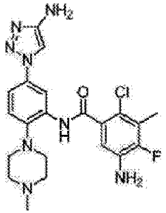

### 5-amino-N-(5-(4-amino-1H-1,2,3-triazol-1-yl)-2-(4-methylpiperazin-1-yl)phenyl)-2-chl oro-4-fluoro-3-methylbenzamide (13)

Compound tert-butyl (1-(3-(5-amino-2-chloro-4-fluoro-3-methylbenzoylamino)-4-(4-methylpiperazin-1-yl)phenyl) -1H-1,2,3-triazol-4-yl)carbamate (12) (1.0 g, 2.2 mmol) was dissolved in 20 mL of dichloromethane, to which 10 mL of trifluoroacetic acid was added. The reaction mixture was stirred at room temperature for 1 h, adjusted to pH = 8-9 with a saturated sodium bicarbonate solution, and extracted with dichloromethane. The organic phase was dried over anhydrous sodium sulfate and spin-dried to obtain a grey solid, with a yield of 87.3%. ¹H NMR (300 MHz, DMSO-*d*₆) *δ* 8.70 (s, 1H), 8.49 (d, *J* = 2.0 Hz, 1H), 8.08 (s, 1H), 7.25 (dd, *J* = 7.5, 2.0 Hz, 1H), 6.80 (d, *J=* 7.5 Hz, 1H), 6.70 (d, *J=* 5.7 Hz, 1H), 5.80 (s, 2H), 4.15 (s, 2H), 3.20 (t, *J* = 5.3 Hz, 4H), 2.98 (t, *J* = 5.2 Hz, 4H), 2.60 (s, 3H), 2.39 (s, 3H). (EI-MS): 459.9 [M + H]⁺.

### Example 14

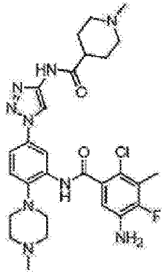

### N-(1-(3-(5-amino-2-chloro-4-fluoro-3-methylbenzoylamino)-4-(4-methylpiperazin-1-yl) phenyl)-1H-1,2,3-triazolopyridin-4-yl)-1-methylpiperidin-4-carboxamide (14)

Compound 5-amino-N-(5-(4-amino-1H-1,2,3-triazol-1-yl)-2-(4-methylpiperazin-1-yl)phenyl)-2-chloro-4 -fluoro-3-methylbenzamide (13) (0.2 g, 0.34 mmol) was dissolved in 5 mL of DMF, to which BOP (0.30 g, 0.68 mmol), triethylamine (0.09 mL, 0.68 mmol) and 1-methylpiperidin-4-carboxylic acid (97.3 mg, 0.68 mmol) were added. The reaction mixture was stirred at room temperature for 4 h, then diluted with 50 mL of ethyl acetate, and washed with a saturated sodium chloride solution to remove DMF. The organic phase was dried over anhydrous sodium sulfate, and spin-dried to remove the organic solvent, thereby obtaining a crude product, which is then purified by silica gel column chromatography (dichloromethane : methanol = 20:1) to obtain an off-white solid, with a yield of 73.9%. ¹H NMR (300 MHz, DMSO-*d*₆) *δ* 8.72- 8.64 (m, 2H), 8.08 (s, 1H), 7.71 (s, 1H), 7.25 (dd, *J* = 7.5, 2.0 Hz, 1H), 6.87 (d, *J =* 7.5 Hz, 1H), 6.69 (d, *J =* 5.7 Hz, 1H), 4.16 (s, 2H), 3.20 (t, *J =* 5.3 Hz, 4H), 3.05-2.95 (m, 6H), 2.60 (s, 3H), 2.50-2.45 (m, 1H), 2.39-2.37 (m, 6H), 2.14-2.09 (m, 2H), 2.05-1.98 (m, 2H), 1.81-1.70 (m, 2H). (EI-MS): 485.1 [M + H]⁺.

### Example 15

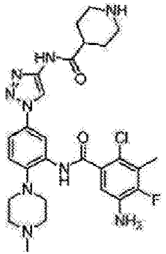

### N-(1-(3-(5-amino-2-chloro-4-fluoro-3-methylbenzoylamino)-4-(4-methylpiperazin-1-yl) phenyl)-1H-1,2,3-triazolopyridin-4-yl)piperidin-4-carboxamide (15)

This example was prepared according to the method in Example 14, wherein 1-methylpiperidin-4-carboxylic acid was replaced with 4-piperidine carboxylic acid. An off-white solid was obtained with a yield of 88.7%. ¹H NMR (300 MHz, DMSO-*d*₆) *δ* 8.70 (s, 1H), 8.55 (d, *J* = 2.0 Hz, 1H), 8.08 (s, 1H), 7.71 (s, 1H), 7.26 (dd, *J* = 7.5, 2.0 Hz, 1H), 6.80 (dd, *J* = 23.8, 6.6 Hz, 2H), 4.15 (s, 2H), 3.27-3.17 (m, 6H), 2.98 (t, *J=* 5.0 Hz, 4H), 2.82-2.65 (m, 3H), 2.60 (s, 3H), 2.39 (s, 3H), 2.03-1.96 (m, 2H), 1.74-1.69 (m, 2H), 1.22 (s, 1H). (EI-MS): 571.1 [M + H]⁺.

### Example 16

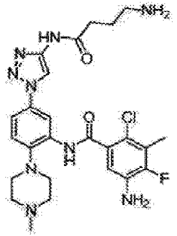

### 5-amino-N-(5-(4-(4-aminobutyrylamino)-1H-1,2,3-triazol-1-yl)-2-(4-methylpiperazin-1-yl)phenyl)-2-chloro-4-fluoro-3-methylbenzamide (16)

This example was prepared according to the method in Example 14, wherein 1-methylpiperidin-4-carboxylic acid was replaced withγ-aminobutyric acid. An off-white solid was obtained with a yield of 88.7%. ¹H NMR (300 MHz, DMSO-*d*₆) *δ* 8.70 (s, 1H), 8.08 (s, 1H), 7.71 (s, 1H), 7.25 (dd, *J=* 7.5, 2.0 Hz, 1H), 6.80 (dd, *J=* 21.4, 6.6 Hz, 2H), 4.17 (s, 2H), 3.20 (t, *J* = 5.1 Hz, 4H), 3.08-3.04 (m, 2H), 2.98 (t, *J* = 5.1 Hz, 4H), 2.60 (s, 3H), 2.50 (t, *J* = 8.2 Hz, 2H), 2.39 (s, 3H), 2.10-2.04 (m, 2H), 1.19 (s, 2H). (EI-MS): 545.0 [M + H]+.

### Example 17

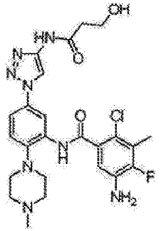

### 5-amino-2-chloro-4-fluoro-N-(5-(4-(3-hydroxypropionylamino)-1H-1,2,3-triazol-1-yl)-2 -(4-methylpiperazin-1-yl)phenyl)-3-methylbenzamide

This example was prepared according to the method in Example 14, wherein 1-methylpiperidin-4-carboxylic acid was replaced with 3-hydroxypropionic acid. An off-white solid was obtained with a yield of 84.9%. ¹H NMR (300 MHz, DMSO-*d*₆) *δ* 8.70 (s, 1H), 8.57 (d, *J* = 2.0 Hz, 1H), 8.08 (s, 1H), 7.71 (s, 1H), 7.25 (dd, *J* = 7.5, 2.0 Hz, 1H), 6.80 (dd, *J=* 21.8, 6.6 Hz, 2H), 4.36 (t, *J=* 5.0 Hz, 1H), 4.17 (s, 2H), 3.83-3.79 (m, 2H), 3.20 (t, *J* = 5.1 Hz, 4H), 2.98 (t, *J=* 5.0 Hz, 4H), 2.60 (s, 3H), 2.39-2.35 (m, 5H). (EI-MS): 532.1 [M + H]+.

### Example 18

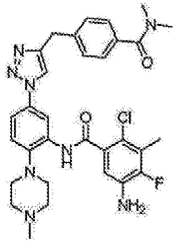

### 5-amino-2-chloro-N-(5-(4-(4-(dimethylaminomethyl)benzyl)-1H-1,2,3-triazol-1-yl)-2-(4 -methylpiperazin-1-yl)phenyl)-4-fluoro-3-methylbenzamide (18)

This example was prepared according to the method in Example 1, wherein methyl propiolate was replaced with N,N-dimethyl-4-(prop-2-yn-1-yl)benzamide. An off-white solid was obtained by three reaction steps. The yield of the three steps was 34.4%. ¹H NMR (300 MHz, DMSO-*d*₆) *δ* 8.70 (s, 1H), 8.49-8.44 (m, 2H), 7.74-7.68 (m, 2H), 7.56-7.50 (m, 2H), 7.26 (dd, *J=* 7.4, 1.9 Hz, 1H), 6.75 (d, *J=* 5.9 Hz, 1H), 4.16 (s, 2H), 3.88 (d, *J=* 1.2 Hz, 2H), 3.20 (t, *J* = 5.1 Hz, 4H), 3.0-2.95 (m, 10H), 2.60 (s, 3H), 2.38 (s, 3H). (EI-MS): 606.1 [M + H]+.

### Example 19

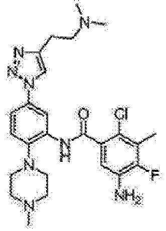

### 5-amino-2-chloro-N-(5-(4-(2-(dimethylamino)ethyl)-1H-1,2,3-triazol-1-yl)-2-(4-methyl piperazin-1-yl)phenyl)-4-fluoro-3-methylbenzamide (19)

This example was prepared according to the method in Example 1, wherein methyl propiolate was replaced with N,N-dimethyl-but-3-yn-1-amine. An off-white solid was obtained by three reaction steps. The yield of the three steps was 31.6%. ¹H NMR (300 MHz, DMSO-*d*₆) *δ* 8.70 (s, 1H), 8.48 (d, *J* = 2.0 Hz, 1H), 8.28 (s, 1H), 7.26 (dd, *J* = 7.5, 2.0 Hz, 1H), 6.82 (d, *J* = 7.5 Hz, 1H), 6.74 (d, *J* = 5.7 Hz, 1H), 4.15 (s, 2H), 3.20 (t, *J* = 5.1 Hz, 4H), 2.98 (t, *J* = 5.0 Hz, 4H), 2.71-2.61 (m, 4H), 2.60 (s, 3H), 2.40-2.39 (m, 9H). (EI-MS): 516.0 [M + H]⁺.

### Example 20

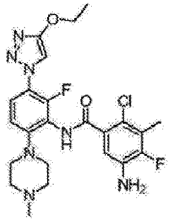

### 5-amino-2-chloro-N-(3-(4-ethoxy-1H-1,2,3-triazol-1-yl)-2-fluoro-6-(4-methylpiperazin-1-yl)phenyl)-4-fluoro-3-methylbenzamide (20)

This example was prepared according to the method in Example 1, wherein 4-fluoro-3-nitroaniline was replaced with 2,4-difluoro-3-nitroaniline and methyl propiolate with ethyl ethynyl ether. An off-white solid was obtained by five reaction steps. The yield of the five steps was 12.8%. ¹H NMR (300 MHz, DMSO-*d*₆) *δ* 8.70 (s, 1H), 7.98 (s, 1H), 7.30 (dd, *J* = 7.5, 5.7 Hz, 1H), 6.74 (d, *J* = 5.7 Hz, 1H), 6.59 (d, *J* = 7.5 Hz, 1H), 4.76-4.72 (m, 2H), 4.15 (s, 2H), 3.20 (t, *J=* 5.1 Hz, 4H), 2.98 (t, *J=* 5.0 Hz, 4H), 2.60 (s, 3H), 2.39 (s, 3H), 1.56 (t, *J=* 8.0 Hz, 3H). (EI-MS): 507.1 [M + H]⁺.

### Example 21

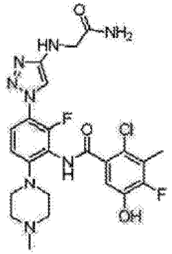

### N-(3-(4-((2-amino-2-oxoethyl)amino)-1H-1,2,3 -triazol-1-yl)-2-fluoro-6-(4-methylpipera zin-1-yl)phenyl)-2-chloro-4-fluoro-5-hydroxy-3-methylbenzamide (21)

This example was prepared according to the method in Example 1, wherein 4-fluoro-3-nitroaniline was replaced with 2,4-difluoro-3-nitroaniline, methyl propiolate with 2-(ethynylamino)acetamide, and 2-chloro-3-methyl-4-fluoro-5-nitrobenzoyl chloride with 2-chloro-3-methyl-4-fluoro-5-hydroxybenzoyl chloride. A white solid was obtained by five reaction steps. The yield of the five steps was 9.7%. ¹H NMR (300 MHz, DMSO-*d*₆) *δ* 8.70 (s, 1H), 8.07 (d, *J=* 10.4 Hz, 2H), 7.34-7.24 (m, 2H), 6.59 (d, *J=* 7.5 Hz, 1H), 5.98 (s, 2H), 5.93 (s, 1H), 4.03 (s, 2H), 3.20 (t, *J* = 5.1 Hz, 4H), 2.98 (t, *J* = 5.0 Hz, 4H), 2.60 (s, 3H), 2.38 (s, 3H). (EI-MS): 536.2 [M + H]⁺.

### Example 22

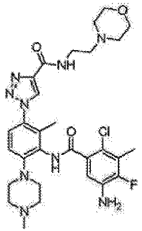

### 1-(3-(5-amino-2-chloro-4-fluoro-3-methylbenzoylamino)-2-methyl-4-(4-methylpiperazi n-1-yl)phenyl)-N-(2-morpholinoethyl)-1H-1,2,3-triazol-4-carboxamide (22)

This example was prepared according to the method in Example 3, wherein 4-fluoro-3-nitroaniline was replaced with 2-fluoro-4-methyl-3-nitroaniline, and dimethylamino hydrochloride with N-(2-aminoethyl)morpholine. An off-white solid was obtained. The yield of the six reaction steps was 8.8%. ¹H NMR (300 MHz, DMSO-*d*₆) *δ* 8.70 (s, 1H), 8.52 (s, 1H), 7.25 (s, 1H), 7.13 (d, *J* = 7.5 Hz, 1H), 6.78 (d, *J* = 5.7 Hz, 1H), 6.72 (d, *J* = 7.5 Hz, 1H), 4.15 (s, 2H), 3.74 (t, *J* = 4.7 Hz, 4H), 3.55-3.53 (m, 2H), 3.20 (t, *J* = 5.1 Hz, 4H), 2.98 (t, *J=* 5.1 Hz, 4H), 2.61-2.59 (m, 5H), 2.51 (t, *J=* 4.7 Hz, 4H), 2.40-2.39 (m, 6H). (EI-MS): 615.1 [M + H]⁺.

### Example 23

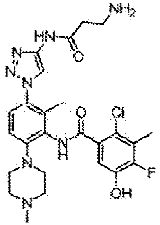

### N-(3-(4-(3-aminopropionylamino)-1H-1,2,3-triazol-1-yl)-2-methyl-6-(4-methylpiperaain -1-yl)phenyl)-2-chloro-4-fluoro-5-hydroxy-3-methylbenzamide (23)

This example was prepared according to the method in Example 14, wherein 4-fluoro-3-nitroaniline was replaced with 2-fluoro-4-methyl-3-nitroaniline, 2-chloro-3-methyl-4-fluoro-5-nitrobenzoyl chloride with 2-chloro-3-methyl-4-fluoro-5-hydroxybenzoyl chloride, and 1-methylpiperidin-4-carboxylic acid with β-alanine. A white solid was obtained by six reaction steps. The yield of the six steps was 7.2%. ¹H NMR (300 MHz, DMSO-*d*₆) *δ* 8.70 (s, 1H), 8.08 (s, 1H), 7.71 (s, 1H), 7.19 (d, *J* = 7.5 Hz, 1H), 7.06 (d, *J* = 5.7 Hz, 1H), 6.72 (d, *J* = 7.5 Hz, 1H), 5.93 (s, 1H), 3.20 (t, *J =* 5.1 Hz, 4H), 3.01-2.93 (m, 4H), 2.93-2.91 (m, 1H), 2.60 (s, 3H), 2.45 (s, 3H), 2.44-2.42 (m, 2H), 2.38 (s, 3H), 1.24 (s, 2H). (EI-MS): 615.1 [M + H]⁺.

### Example 24

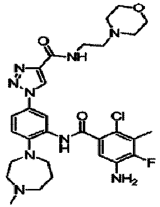

### 1-(3-(5-amino-2-chloro-4-fluoro-3-methylbenzoylamino)-4-(4-methyl-1,4-diazepan-1-yl )phenyl)-N-(2-morpholinoethyl)-1H-1,2,3-triazol-4-carboxamide (24)

This example was prepared according to the method in Example 3, wherein N-methylpiperazine was replaced with N-methylhomopiperazine, and dimethylamino hydrochloride with N-(2-aminoethyl)morpholine. An off-white solid was obtained. The yield of the six reaction steps was 7.3%. ¹H NMR (300 MHz, DMSO-*d*₆) *δ* 8.79 (s, 1H), 8.70 (s, 1H), 7.31 (dd, *J* = 7.5, 2.0 Hz, 1H), 7.25 (s, 1H), 6.83 (d, *J* = 7.5 Hz, 1H), 6.70 (d, *J* = 5.7 Hz, 1H), 4.16 (s, 2H), 3.74 (t, *J* = 4.7 Hz, 4H), 3.60 (t, *J* = 4.8 Hz, 2H), 3.55-3.53 (m, 2H), 3.46-3.44 (m, 2H), 2.91-2.89 (m, 2H), 2.61-2.58 (m, 4H), 2.52-2.50 (m, 4H), 2.39 (s, 3H), 2.31 (s, 3H), 1.64-1.60 (m, 2H). (EI-MS): 615.1 [M + H]⁺.

### Example 25

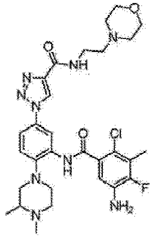

### 1-3-(5-amino-2-chloro-4-fluoro-3-methylbenzoylamino)-4-(3,4-dimethylpiperazin-1-yl) phenyl)-N-(2-morpholinoethyl)-1H-1,2,3-triazol-4-carboxamide (25)

This example was prepared according to the method in Example 3, wherein N-methylpiperazine was replaced with 1,2-dimethylpiperazine, and dimethylamino hydrochloride with N-(2-aminoethyl)morpholine. An off-white solid was obtained. The yield of the six reaction steps was 7.3%. ¹H NMR (300 MHz, DMSO-*d*₆) *δ* 8.80 (s, 1H), 8.70 (s, 1H), 8.54 (d, *J* = 2.0 Hz, 1H), 7.31 (dd, *J* = 7.5, 2.0 Hz, 1H), 7.25 (s, 1H), 6.81 (d, *J* = 7.5 Hz, 1H), 6.70 (d, *J =* 5.7 Hz, 1H), 4.18-4.04 (m, 7H), 3.66-3.53 (m, 2H), 3.35-3.31 (m, 1H), 3.20-3.07 (m, 4H), 2.93-2.87 (m, 1H), 2.73-2.58 (m, 2H), 2.51-2.37 (m, 7H), 2.34-2.20 (m, 3H), 1.16 (d, *J* = 6.8 Hz, 3H). (EI-MS): 615.1 [M + H]⁺.

## Claims

1. A compound of general formula (I) or a pharmaceutically acceptable salt thereof: 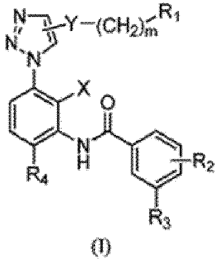 wherein X is hydrogen, methyl, methoxy or halogen;
Y is -CH₂-, -O-, -S-, -CO-, -CH₂O-, -NR₅-, -CONR₆- or -NR₇CO-, wherein R₅, R₆, and R₇ are each independently hydrogen, C₁-C₄ alkyl, C₁-C₄ haloalkyl, phenyl or substituted phenyl, wherein the substituent is halogen, C₁-C₄ alkyl, C₁-C₄ alkoxy, amino, hydroxyl, mercapto, carboxyl, cyano, trifluoromethyl or imidazolyl;
m is 0-6;
R₁ is hydrogen, amino, hydroxyl, mercapto, carboxyl, cyano, -CONH₂, C₁-C₄ alkyl, C₁-C₄ alkoxy, phenyl, substituted phenyl, substituted or unsubstituted nitrogen- or oxygen-containing 3 to 7 membered heterocyclic ring, -NR₈COR₉, -CONR₁₀R₁₁ or -NR₁₀R₁₁, wherein R₈ is hydrogen, C₁-C₄ alkyl, C₁-C₄ haloalkyl, phenyl or substituted phenyl, R₉ is amino, hydroxyl, C₁-C₄ alkyl, C₁-C₄ alkoxy, phenyl or substituted phenyl, substituted or unsubstituted nitrogen- or oxygen-containing 3 to 7 membered heterocyclic ring, R₁₀ and R₁₁ are independently hydrogen, C₁-C₄ alkyl, phenyl or substituted phenyl, substituted or unsubstituted nitrogen- or oxygen-containing 3 to 7 membered heterocyclic ring, or R₁₀ and R₁₁ are connected to form nitrogen- or oxygen-containing 3 to 7 membered heterocyclic ring, wherein the substituent is halogen, C₁-C₄ alkyl, C₁-C₄ alkoxy, amino, hydroxyl, mercapto, carboxyl, cyano, trifluoromethyl or imidazolyl;
R₂ is halogen, C₁-C₄ alkyl, C₁-C₄ alkoxy, trifluoromethyl, nitro or cyano for disubstitution or trisubstitution;
R₃ is amino, methylamino, aminomethyl, hydroxyl, hydroxymethyl, mercapto or - CONH₂;
R₄ is N-methylpiperazine, 1,2-dimethylpiperazine or N-methylhomopiperazine.

2. The compound of claim 1 or a pharmaceutically acceptable salt thereof, wherein X is hydrogen, fluorine, chlorine or methyl.

3. The compound of claim 1 or a pharmaceutically acceptable salt thereof, wherein Y is - NR₅-, -CONR₆- or -NR₇CO-; R₅, R₆ and R₇ are each independently hydrogen, methyl, ethyl, propyl, cyclopropyl or isopropyl.

4. The compound of claim 1 or a pharmaceutically acceptable salt thereof, wherein Y is - NR₅-, -CONR₆- or -NR₇CO-; R₅, R₆ and R₇ are each independently substituted phenyl, wherein the substituent is methyl, ethyl, isopropyl, t-butyl, cyclopropyl, methoxy, cyano, halogen, trifluoromethyl or imidazolyl.

5. The compound of claim 1 or a pharmaceutically acceptable salt thereof, wherein the substituted or unsubstituted nitrogen- or oxygen-containing 3 to 7 membered heterocyclic ring is aziridine, azetidine, tetrahydropyrrole, piperidine, hexamethyleneimine, lactam, tetrahydrofuran, tetrahydropyran, morpholine, 1,4-oxazepane, hexahydropyridazine, imidazoline, pyrazolidine, piperazine, wherein the substituent is methyl, ethyl, phenyl, hydroxyl, amino, hydroxymethyl, or aminomethyl, and the substituent is halogen, C₁-C₄ alkyl, C₁-C₄ alkoxy, amino, hydroxyl, mercapto, carboxyl, cyano, or trifluoromethyl.

6. The compound of claim 1 or a pharmaceutically acceptable salt thereof, wherein R₁ is -NR₈COR₉, -CONR₁₀R₁₁ or -NR₁₀R₁₁, wherein R₈, R₉, R₁₀ and R₁₁ are C1-C4 alkyl.

7. The compound of claim 1 or a pharmaceutically acceptable salt thereof, wherein R₂ is a substituent for trisubstitution and is fluorine, chlorine, bromine, methyl, methoxy, nitro, trifluoromethyl or cyano.

8. A compound or a pharmaceutically acceptable salt thereof, wherein the compound is:

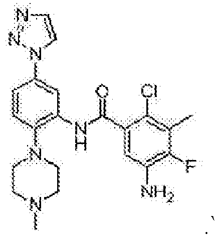

9. A pharmaceutical composition comprising the compound of any one of claims 1 to 8 or a pharmaceutically acceptable salt thereof and a pharmaceutically acceptable carrier.

10. The compound of any one of claims 1 to 8 or a pharmaceutically acceptable salt thereof, for use as a medicament for the treatment of acute leukemia.

11. The compound for use of claim 10, wherein the acute leukemia is acute leukemia with MLL 1 gene rearrangement.

12. A pharmaceutical composition for use in the treatment of acute leukemia comprising a compound of any of claims 1-8 or a pharmaceutically acceptable salt thereof and a pharmaceutically acceptable carrier.

## Patentansprüche

1. Verbindung der allgemeinen Formel (I) oder ein pharmazeutisch annehmbares Salz davon: 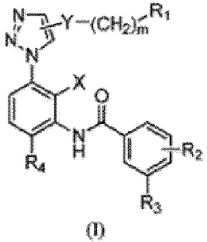 worin X Wasserstoff, Methyl, Methoxy oder Halogen ist;
Y -CH₂-, -O-, -S-, -CO-, -CH₂O-, -NR₅-, -CONR₆- oder -NR₇CO- ist, worin R₅, R₆ und R₇ jeweils unabhängig Wasserstoff, C₁₋₄-Alkyl, C₁₋₄-Halogenalkyl, Phenyl oder substituiertes Phenyl sind, wobei der Substituent Halogen, C₁₋₄-Alkyl, C₁₋₄-Alkoxy, Amino, Hydroxyl, Mercapto, Carboxyl, Cyano, Trifluormethyl oder Imidazolyl ist;
m = 0 bis 6 ist;
R₁ Wasserstoff, Amino, Hydroxyl, Mercapto, Carboxyl, Cyano, -CONH₂, C₁₋₄-Alkyl, C₁₋₄-Alkoxy, Phenyl, substituiertes Phenyl, ein substituierter oder unsubstituierter, Stickstoff- oder Sauerstoff-hältiger, 3- bis 7-gliedriger heterozyklischer Ring, -NR₈COR₉, -CONR₁₀R₁₁ oder -NR₁₀R₁₁ ist, worin R₈ Wasserstoff, C₁₋₄-Alkyl, C₁₋₄-Halogenalkyl, Phenyl oder substituiertes Phenyl ist, R₉ Amino, Hydroxyl, C₁₋₄-Alkyl, C₁₋₄-Alkoxy, Phenyl oder substituiertes Phenyl oder ein substituierter oder unsubstituierter, Stickstoff- oder Sauerstoff-hältiger, 3- bis 7-gliedriger heterozyklischer Ring ist, R₁₀ und R₁₁ jeweils unabhängig Wasserstoff, C₁₋₄-Alkyl, Phenyl oder substituiertes Phenyl, ein substituierter oder unsubstituierter, Stickstoff- oder Sauerstoff-hältiger, 3- bis 7-gliedriger heterozyklischer Ring sind oder R¹⁰ und R¹¹ miteinander zu einem Stickstoff- oder Sauerstoff-hältigen, 3- bis 7-gliedrigen heterozyklischen Ring verbunden sind, wobei der Substituent Halogen, C₁₋₄-Alkyl, C₁₋₄-Alkoxy, Amino, Hydroxyl, Mercapto, Carboxyl, Cyano, Trifluormethyl oder Imidazolyl ist;
R₂ Halogen, C₁₋₄-Alkyl, C₁₋₄-Alkoxy, Trifluormethyl, Nitro oder Cyano zur Disubstitution oder Trisubstitution ist;
R₃ Amino, Methylamino, Aminomethyl, Hydroxyl, Hydroxymethyl, Mercapto oder -CONH₂ ist; und
R₄ N-Methylpiperazin, 1,2-Dimethylpiperazin oder N-Methylhomopiperazin ist.

2. Verbindung nach Anspruch 1 oder ein pharmazeutisch annehmbares Salz davon, worin X Wasserstoff, Fluor, Chlor oder Methyl ist.

3. Verbindung nach Anspruch 1 ein oder pharmazeutisch annehmbares Salz davon, worin Y -NR₅-, -CONR₆- oder -NR₇CO- ist; und R₅, R₆ und R₇ jeweils unabhängig Wasserstoff, Methyl, Ethyl, Propyl, Cyclopropyl oder Isopropyl sind.

4. Verbindung nach Anspruch 1 oder ein pharmazeutisch annehmbares Salz davon, worin Y -NR₅-, -CONR₆- oder-NR₇CO- ist; R₅, R₆ und R₇ jeweils unabhängig substituiertes Phenyl sind, wobei der Substituent Methyl, Ethyl, Isopropyl, t-Butyl, Cyclopropyl, Methoxy, Cyano, Halogen, Trifluormethyl oder Imidazolyl ist.

5. Verbindung nach Anspruch 1 oder ein pharmazeutisch annehmbares Salz davon, worin der substituierte oder unsubstituierte, Stickstoff- oder Sauerstoff-hältige, 3- bis 7-gliedrige heterozyklische Ring Aziridin, Azetidin, Tetrahydropyrrol, Piperidin, Hexamethylenimin, Lactam, Tetrahydrofuran, Tetrahydropyran, Morpholin, 1,4-Oxazepan, Hexahydropyridazin, Imidazolin, Pyrazolidin oder Piperazin ist, wobei der Substituent Methyl, Ethyl, Phenyl, Hydroxyl, Amino, Hydroxymethyl oder Aminomethyl ist und der Substituent Halogen, C₁₋₄-Alkyl, C₁₋₄-Alkoxy, Amino, Hydroxyl, Mercapto, Carboxyl, Cyano oder Trifluormethyl ist.

6. Verbindung nach Anspruch 1 oder ein pharmazeutisch annehmbares Salz davon, worin R₁ -NR₈COR₉, CONR₁₀R₁₁ oder-NR₁₀R₁₁ ist, worin R₈, R₉, R₁₀ und R₁₁ C₁₋₄-Alkyl sind.

7. Verbindung nach Anspruch 1 oder ein pharmazeutisch annehmbares Salz davon, worin R₂ ein Substituent zur Trisubstitution ist und Fluor, Chlor, Brom, Methyl, Methoxy, Nitro, Trifluormethyl oder Cyano ist.

8. Verbindung oder ein pharmazeutisch annehmbares Salz davon, wobei die Verbindung die folgende ist:

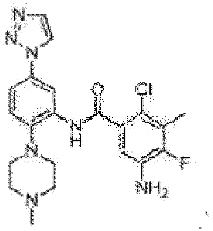

9. Pharmazeutische Zusammensetzung, die eine Verbindung nach einem der Ansprüche 1 bis 8 oder ein pharmazeutisch annehmbares Salz davon und einen pharmazeutisch annehmbaren Träger umfasst.

10. Verbindung nach einem der Ansprüche 1 bis 8 oder ein pharmazeutisch annehmbares Salz davon zur Verwendung als Medikament zur Behandlung von akuter Leukämie.

11. Verbindung zur Verwendung nach Anspruch 10, wobei die akute Leukämie akute Leukämie mit MILL1-Gen-Umordnung ist.

12. Pharmazeutische Zusammensetzung zur Verwendung bei der Behandlung von akuter Leukämie, die eine Verbindung nach einem der Ansprüche 1 bis 8 oder ein pharmazeutisch annehmbares Salz davon und einen pharmazeutisch annehmbaren Träger umfasst.

## Revendications

1. Composé de formule générale (I) ou un sel pharmaceutiquement acceptable de celui-ci : 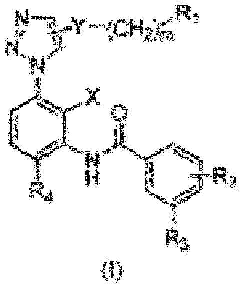 dans lequel X est un hydrogène, un méthyle, un méthoxy ou un halogène ;
Y est -CH₂-, -O-, -S-, -CO-, -CH₂O-, -NR₅-, -CONR₆- ou -NR₇CO-, dans lequel R₅, R₆ et R₇ sont chacun indépendamment de l'hydrogène, un alkyle en C₁-C₄, un haloalkyle en C₁-C₄, un phényle ou un phényle substitué, dans lequel le substituant est un halogène, un alkyle en C₁-C₄, un alcoxy en C₁-C₄, un amino, un hydroxyle, un mercapto, un carboxyle, un cyano, un trifluorométhyle ou un imidazolyle ;
m est 0 à 6 ;
R₁ est un hydrogène, un amino, un hydroxyle, un mercapto, un carboxyle, un cyano, -CONH₂, un alkyle en C₁-C₄, un alcoxy en C₁-C₄, un phényle, un phényle substitué, un cycle hétérocyclique de 3 à 7 chaînons contenant de l'azote ou de l'oxygène substitué ou non substitué, -NR₈COR₉, -CONR₁₀R₁₁ ou -NR₁₀R₁₁, dans lequel R₈ est un hydrogène, un alkyle en C₁-C₄, un haloalkyle en C₁-C₄, un phényle ou un phényle substitué, R₉ est un amino, un hydroxyle, un alkyle en C₁-C₄, un alcoxy en C₁-C₄, un phényle ou un phényle substitué, un cycle hétérocyclique de 3 à 7 chaînons contenant de l'azote ou de l'oxygène substitué ou non substitué, R₁₀ et R₁₁ sont indépendamment un hydrogène, un alkyle en C₁-C₄, un phényle ou un phényle substitué, un cycle hétérocyclique de 3 à 7 chaînons contenant de l'azote ou de l'oxygène substitué ou non substitué, ou R₁₀ et R₁₁ sont connectés pour former un cycle hétérocyclique de 3 à 7 chaînons contenant de l'azote ou de l'oxygène, dans lequel le substituant est un halogène, un alkyle en C₁-C₄, un alcoxy en C₁-C₄, un amino, un hydroxyle, un mercapto, un carboxyle, un cyano, un trifluorométhyle ou un imidazolyle ;
R₂ est un halogène, un alkyle en C₁-C₄, un alcoxy en C₁-C₄, un trifluorométhyle, un nitro ou un cyano pour une disubstitution ou une trisubstitution ;
R₃ est un amino, un méthylamino, un aminométhyle, un hydroxyle, un hydroxyméthyle, un mercapto ou -CONH₂ ;
R₄ est une N-méthylpipérazine, une 1,2-diméthylpipérazine ou une N-méthylhomopipérazine.

2. Composé selon la revendication 1 ou un sel pharmaceutiquement acceptable de celui-ci, dans lequel X est de l'hydrogène, du fluor, du chlore ou du méthyle.

3. Composé selon la revendication 1 ou un sel pharmaceutiquement acceptable de celui-ci, dans lequel Y est -NR₅-, -CONR₆- ou -NR₇CO- ; R₅, R₆ et R₇ sont chacun indépendamment un hydrogène, un méthyle, un éthyle, un propyle, un cyclopropyle ou un isopropyle.

4. Composé selon la revendication 1 ou un sel pharmaceutiquement acceptable de celui-ci, dans lequel Y est -NR₅-, -CONR₆- ou -NR₇CO- ; R₅, R₆ et R₇ sont chacun indépendamment un phényle substitué, dans lequel le substituant est un méthyle, un éthyle, un isopropyle, un t-butyle, un cyclopropyle, un méthoxy, un cyano, un halogène, un trifluorométhyle ou un imidazolyle.

5. Composé selon la revendication 1 ou un sel pharmaceutiquement acceptable de celui-ci, dans lequel le cycle hétérocyclique à 3 à 7 chaînons contenant de l'azote ou de l'oxygène substitué ou non substitué est l'aziridine, l'azétidine, le tétrahydropyrrole, la pipéridine, l'hexaméthylèneimine, le lactame, le tétrahydrofurane, le tétrahydropyrane, la morpholine, le 1,4-oxazépane, l'hexahydropyridazine, l'imidazoline, la pyrazolidine, la pipérazine, dans lequel le substituant est un méthyle, un éthyle, un phényle, un hydroxyle, un amino, un hydroxyméthyle ou un aminométhyle, et le substituant est un halogène, un alkyle en C₁-C₄, un alcoxy en C₁-C₄, un amino, un hydroxyle, un mercapto, un carboxyle, un cyano ou un trifluorométhyle.

6. Composé selon la revendication 1 ou un sel pharmaceutiquement acceptable de celui-ci, dans lequel R₁ est -NR₈COR₉, -CONR₁₀R₁₁ ou -NR₁₀R₁₁, dans lequel R₈, R₉, R₁₀ et R₁₁ sont un alkyle en C₁-C₄.

7. Composé selon la revendication 1 ou un sel pharmaceutiquement acceptable de celui-ci, dans lequel R₂ est un substituant pour une trisubstitution et est le fluor, le chlore, le brome, le méthyle, le méthoxy, le nitro, le trifluorométhyle ou le cyano.

8. Composé ou un sel pharmaceutiquement acceptable de celui-ci, dans lequel le composé est :

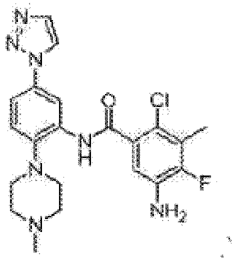

9. Composition pharmaceutique comprenant le composé selon l'une quelconque des revendications 1 à 8, ou un sel pharmaceutiquement acceptable de celui-ci, et un support pharmaceutiquement acceptable.

10. Composé selon l'une quelconque des revendications 1 à 8 ou un sel pharmaceutiquement acceptable de celui-ci, à utiliser en tant que médicament pour le traitement de la leucémie aiguë.

11. Composé à utiliser selon la revendication 10, dans lequel la leucémie aiguë est une leucémie aiguë avec un réarrangement du gène MLL1.

12. Composition pharmaceutique à utiliser dans le traitement de la leucémie aiguë comprenant un composé selon l'une quelconque des revendications 1 à 8, ou un sel pharmaceutiquement acceptable de celui-ci, et un support pharmaceutiquement acceptable.
